# EUROPEAN PATENT APPLICATION

(11) **EP 2 462 929 A1**
(43) Date of publication of application: **13.06.2012**
(21) Application number: 11009697.1
(22) Date of filing: 08.12.2011
(51) Int. Cl.: A61K 9/70, A61K 9/48, A61K 39/36, A61P 37/08

(54) **Sheet-form preparation and method for producing the same**

(30) Priority: 10.12.2010 JP 2010276189; 15.04.2011 JP 2011091327
(71) Applicant: Nitto Denko Corporation, Osaka 567-8680 (JP)
(72) Inventor: Daisuke, Asari, Ibaraki-shi Osaka 567-8680 (JP); Takuya, Shishido, Ibaraki-shi Osaka 567-8680 (JP); Mitsuhiko, Hori, Ibaraki-shi Osaka 567-8680 (JP); Toshihiko, Okazaki, Ibaraki-shi Osaka 567-8680 (JP); Tatsuya, Konishi, Ibaraki-shi Osaka 567-8680 (JP)
(74) Representative: Müller-Boré & Partner Patentanwälte

(57) **Abstract**

The present invention provides a sheet-form preparation that can be easily dissolved intraorally, allows the dissolution time thereof to be easily controlled, and can stably contain an allergenic protein from cedar pollen.

The sheet-form preparation contains water, gelatin, an allergenic protein from cedar pollen, and a stabilizing agent of the allergenic protein from cedar pollen.

## Description

The present invention relates to a sheet-form preparation containing an allergenic protein from cedar pollen. More specifically, the present invention relates to a sheet-form preparation that may easily dissolve intraorally in hyposensitization therapy for cedar pollinosis; and a production method for the sheet-form preparation.

The current situation for treatments against allergy diseases, such as pollen allergies, is that most of them are symptomatic treatments using antihistamines; in recent years, hyposensitization therapies are gathering attention as therapeutic methods that can completely cure allergy diseases.
Since hyposensitization therapy in general requires a long-period administration of about two to three years, it is thought that a dosage form is necessary that further improves the QOL (quality of life) of the caregiver and the patient.

Currently, dosage forms used in specific hyposensitization therapy are, for the most part, injectables for subcutaneous injections.
In specific hyposensitization therapy by subcutaneous injection, however, some problems exist such as danger of anaphylactic shock, necessity of administration by a healthcare professional, necessity of frequent hospital visits over a long period, pain due to injection, and storage under refrigeration.

In addition, Patent Document 1, Patent Document 2, and Patent Document 3 disclose preparations in film shape in which a drug is dispersed or dissolved in a water-soluble polymer, for instance, which also refer to plant pollen and gelatin.

However, conventional preparations in film shape contain water-soluble polymers as an ingredient of the base material. Thus, in order to dissolve the preparations in the mouth cavity, a large quantity of saliva is required, and dissolving of the preparations may take a long period. In addition, the Patent Documents disclose a production method for such conventional preparations in film shape comprising dissolving the water-soluble polymer using water as a solvent, dispersing or dissolving a plant pollen thereinto, and heat-drying it. In such a method, particularly in the case that a plant pollen that is vulnerable to heat has been dispersed or the like, a decrease in the activity of the plant pollen due to heat is a concern. In the case that the plant pollen is an extract in liquid form, the preparation in film form may be disadvantageously dissolved, so that maintaining of a given shape may be difficult. Further, it is also difficult to appropriately control the time of dissolution in the mouth cavity. Thus, these preparations require additional improvements to be used in hyposensitization therapy of allergy disease patients.

Patent Document 1: JP-T 2005-511522
Patent Document 2: JP-T 2007-500252
Patent Document 3: JP-T 2009-507854

In view of the current situation described above, the present invention aims to provide a sheet-form preparation that can be easily dissolved intraorally, that can allow the dissolution time thereof to be easily controlled, and that can stably contain an allergenic protein from cedar pollen; and a production method for the sheet-form preparation.

As a result of earnest studies in order to solve the problems described above, the present inventors have discovered that a sheet-form preparation containing, as the base material, gelatin that gels at ordinary temperatures, that readily dissolves at temperatures of about the body temperature, and that contributes to the stabilization of an allergenic protein from cedar pollen that is vulnerable to heat, and containing a stabilizing agent that increases the stability of the allergenic protein from cedar pollen, has clearly improved preparation characteristics and storage stability that are appropriate for hyposensitization therapy by the intraoral route, compared to conventional products. They have also found that such a sheet-form preparation can be prepared without heating and is excellent in the stability of the allergenic protein from cedar pollen during production. Thereby, the present inventors have reached completion of the present invention.

That is, the present invention relates to a sheet-form preparation comprising water, gelatin, an allergenic protein from cedar pollen, and a stabilizing agent of the allergenic protein from cedar pollen.
The sheet-form preparation of the present invention is preferably provided for intraoral hyposensitization therapy use.
The gelatin preferably has a property of not gelling at 32°C when turned into an aqueous solution at a concentration of 10 wt%.
In the sheet-form preparation of the present invention, the allergenic protein from cedar pollen is preferably provided in a liquid or solid form containing this protein.
The amount of gelatin is preferably 2 to 40 wt% based on the total weight.
The sheet-form preparation of the present invention preferably has a thickness within the range of 30 to 5000 µm and a planar surface area within the range of 0.5 to 6.0 cm² .
The stabilizing agent preferably contains at least one selected from the group consisting of sugars, sugar alcohols, and sugar fatty acids.
The sheet-form preparation of the present invention preferably further comprises polyethylene glycol or a derivative thereof; preferably, it further comprises crystalline cellulose.
In addition, the present invention relates to a method for producing the sheet-form preparation of the present invention. The production method comprises the steps of: preparing a mixed solution by mixing water, gelatin, an allergenic protein from cedar pollen, and a stabilizing agent of the allergenic protein from cedar pollen; and forming a thin film using the mixed solution. In the production method, the water content in the obtained sheet-form preparation is adjusted by adjusting the amount of water to be added in the step of preparing the mixed solution or by nonthermally drying the thin film after the step of forming the thin film.
Hereinafter, the present invention will be described in detail.

The sheet-form preparation of the present invention contains water, gelatin, an allergenic protein from cedar pollen, and a stabilizing agent of the allergenic protein from cedar pollen.
The sheet-form preparation of the present invention having such a composition is used adequately for intraoral hyposensitization therapy, which requires control of sensitized time, and is particularly suited for sublingual hyposensitization therapy. In addition, since the sheet-form preparation of the present invention contains gelatin and a specific stabilizing agent, the allergenic protein from cedar pollen can be stably maintained.

The thickness of the sheet-form preparation of the present invention is not particularly limited, and it is preferably 30 to 5000 µm. A film having a thickness of less than 30 µm may suffer problems in film strength and product handleability, while a film having a thickness of higher than 5000 µm may cause discomfort when administering in the mouth cavity, in particular under the tongue.
In addition, the size of the sheet-form preparation of the present invention is not particularly limited, and it is preferable that the planar surface area is within the range of 0.5 to 6.0 cm². A film having a surface area of less than 0.5 cm² may cause difficulty in handling when pinch-administering the sheet-form preparation, while a film having a surface area of higher than 6.0 cm² may not be perfectly inserted in the mouth cavity, in particular under the tongue.
In addition, the planar shape of the sheet-form preparation of the present invention is not particularly limited. Examples thereof include rectangles such as oblongs and squares, polygons such as pentagons, circles, ellipses, and the like. Polygons referred to herein include, in addition to complete polygons, shapes having a slight curve in an angular portion.
The term "sheet-form" herein is a concept that includes "film-form".

The sheet-form preparation of the present invention contains gelatin.
The gelatin is an ingredient that constitutes the base material of the sheet-form preparation of the present invention, and has a sheet shape-forming ability and edibility.
By containing such a gelatin, the sheet-form preparation of the present invention gels at ordinary temperature and can be dissolved easily at a temperature of about the body temperature inside the mouth cavity.
The term "edibility" herein means that the gelatin is perorally administrable and is pharmaceutically acceptable.

The gelatin is preferably in a grade referred to as water-soluble gelatin. Using the water-soluble gelatin enables to prepare the sheet-form preparation of the present invention at near ordinary temperature and to secure stability of the allergenic protein from cedar pollen described below during production.
The term "water-soluble gelatin" herein indicates a gelatin such that 1 g of gelatin dissolves in 20 mL of water at ordinary temperature (30°C).

Further, the gelatin preferably has a characteristic such that it does not gel at 32°C when turned into an aqueous solution at a concentration of 10 wt%. This is because some gelatins having such a characteristic may sufficiently provide the effects of the present invention depending on the molecular weight thereof and the amount of hydroxyproline therein even if they are beyond the grade of water-soluble gelatins.
The gelatin preferably gels at near 5°C.

Examples of the gelatin used in the sheet-form preparation of the present invention include those prepared by enzymatically decomposing and extracting proteins contained in animal skin or bone, such as those obtained by acid-treating or alkali-treating proteins derived from pigs, cattle, and fish.
The above-mentioned gelatin is particularly preferably a gelatin derived from fish or pigs from the viewpoints of being producible at ordinary temperature during production and stability of the allergenic protein from cedar pollen during production.
From such viewpoints, any gelatin may be adequate as long as it contains hydroxyproline in the amino acid composition in an amount of 5.2 to 9.2 mol% and it has an average molecular weight of exceeding 90,000. Examples of such a gelatin include those derived from fishes such as salmon-derived gelatin (hydroxyproline amount in amino acid composition: 5.4 mol%), carp-derived gelatin (hydroxyproline amount in amino acid composition: 7.6 mol%), and tilapia-derived gelatin (hydroxyproline amount in amino acid composition: 8.0 mol%). Particularly preferable is tilapia-derived gelatin.

Here, the above-mentioned amino acid composition is obtained by analysis wherein gelatin is hydrolyzed, then separated by ion-exchange chromatography, and detected by ninhydrin.
Specific examples of the amount of hydroxyproline in the amino acid composition (mol%) obtained by the above-described method are as follows.
Fowl: 10.8 mol%
Ostrich: 10.4 mol%
Mouse: 8.7 mol%
Pig: 9.4 mol%
Cattle: 9.5 mol%

In addition, any gelatin may be preferable regardless of the amount of hydroxyproline in the amino acid composition as long as it has an average molecular weight of 50,000 to 90,000.
The term "average molecular weight" herein means a weight average molecular weight measured by gel-filtration chromatography analysis.
Further, the average molecular weight herein means a molecular weight of each polypeptide chain monomer, not the molecular weight of the polypeptide chain trimer, of gelatin.

In the sheet-form preparation of the present invention, the amount of the gelatin is preferably 2 to 40 wt%, and more preferably 3 to 30 wt%, based on the total weight of the sheet-form preparation of the present invention. If the amount is less than 2 wt%, the gelatin may not gel at ordinary temperature. On the other hand, if the amount is in excess of 40 wt%, the solubility of the sheet-form preparation of the present invention in the mouth cavity becomes extremely low, which may cause a problem during use.

In addition to the above-mentioned gelatin which is an edible polymer, the sheet-form preparation of the present invention may also contain a suitable amount of an edible polymer that is soluble only in water or an edible polymer that dissolves neither in water nor in an organic solvent (hereafter, these are collectively referred to as other edible polymers) in combination, to the extent that they do not inhibit the effects of the present invention.

Examples of the other edible polymers include synthetic polymers such as polyethylene glycol, polyvinyl alcohol, carboxyvinyl polymer, hydroxypropylmethyl cellulose, hydroxyethyl cellulose, methyl cellulose, ethyl cellulose, hydroxypropyl cellulose with a low substitution degree, crystalline cellulose, sodium carboxymethyl cellulose, calcium carboxymethyl cellulose, carboxymethyl cellulose, and sodium carboxymethyl starch; polymers obtained from natural products such as dextran, casein, guar gum, xanthan gum, tragacanth gum; acacia gum, gum arabic, gellan gum, and starch. Each of these other edible polymers can be used alone or two or more of these can be used in combination.
The amount of the other edible polymers is preferably 0.1 to 10 wt% based on the total weight of the sheet-form preparation of the present invention.

Preferable among the other edible polymers are polyethylene glycol or a derivative thereof, and crystalline cellulose; particularly preferable among these is polyethylene glycol due to its stabilization effect on the allergenic protein from cedar pollen described below.
In addition, from the viewpoint of stabilization effect on the conservation of allergenic protein from cedar pollen, the molecular weight of the polyethylene glycol is preferably 20,000 or less. For stabilized physical properties of the sheet-form preparation of the present invention, the molecular weight of the polyethylene glycol is preferably 1,000 or greater. The term "molecular weight" herein means a weight average molecular weight measured by gel-filtration chromatography analysis.

The sheet-form preparation of the present invention contains an allergenic protein from cedar pollen.
Examples of the allergenic protein from cedar pollen include those containing, as an effective ingredient, one or more selected from the group consisting of proteins that have antigenicity specifically reacting with an antibody of a person having an allergy disease and that are extracted from cedar pollen, and proteins that have high homology with the above proteins in amino acid level.
In the sheet-form preparation of the present invention, the allergenic protein from cedar pollen may be in liquid form or a solid containing these. Here, one in liquid form is referred to as a cedar pollen extract, and a cedar pollen extract in liquid form has been so far difficult to solidify from the viewpoints of extraction thereof and of stability of the allergenic protein from cedar pollen. In the sheet-form preparation of the present invention, however, a cedar pollen extract even in liquid form can be solidified satisfactorily.

Examples of the proteins having antigenicity extracted from cedar pollen include proteins contained in cedar pollen capable of inducing the production of cedar pollen-specific IgE antibodies.
These proteins comprise major allergenic proteins from cedar pollen and minor allergenic proteins from cedar pollen. The major allergenic proteins from cedar pollen are components to which a majority of patients are strongly sensitized among a number of cedar pollen extracts that are included in pollen, while the minor allergenic proteins from cedar pollen are components to which only a portion of the patients are sensitized.
Particularly preferable as cedar pollen extracts are Cryj1 and Cryj2, which are major allergenic proteins from cedar pollen, and a mixture thereof. Also preferable are cedar pollen extracts as they are, which are cedar pollen extraction solutions containing not only the Cryj1 and Cryj2 but also minor allergenic proteins from cedar pollen, or those in diluted form.
Torii Pharmaceutical Co., Ltd. markets, as commercially available pharmaceutical products, standardized cedar pollen extract treatment extract "Torii" cedar pollen 200 JAU/mL and standardized cedar pollen extract treatment extract "Torii" cedar pollen 2000 JAU/mL, which correspond to the above cedar pollen extracts. In the sheet-form preparation of the present invention, the above-mentioned pharmaceutical products or the above-mentioned stock solutions may be used.
The term "JAU" mentioned above is an abbreviation for "Japanese allergy units" and means the potency of an allergenic protein from cedar pollen standardized with Cryj1, which is a major allergenic protein from cedar pollen. In addition, cedar pollen extract-Cj manufactured by LSL Co., Ltd., for example, may be also used.

The amount of the allergenic protein from cedar pollen depends on the properties and the like thereof. In general, the amount is preferably 1 × 10⁻¹⁰ to 80 wt% to the total weight of the sheet-form preparation of the present invention. If the amount is less than 1 × 10⁻¹⁰ wt%, the preparation may not be suitable for hyposensitization therapy from the viewpoint of clinical effects. If the amount is in excess of 80 wt%, the strength of the sheet-form preparation of the present invention may be reduced noticeably, likely causing problems in the ability to maintain the shape. The amount of the allergenic protein from cedar pollen is more preferably in the range of 1 × 10⁻⁷ to 1 × 10⁻¹ wt%. An amount in this range probably leads to optimal clinical effects in practice.
From the viewpoints of the amount of Cryj1, which is one of the major allergenic proteins from cedar pollen, and of clinical effects, the sheet-form preparation preferably contains the allergenic protein from cedar pollen at 0.01 ng to 1 mg per sheet.

The sheet-form preparation of the present invention contains a stabilizing agent of the allergenic protein from cedar pollen described above.
Examples of the stabilizing agent include those containing at least one selected from the group consisting of sugars, sugar alcohols, and sugar fatty acids.
Examples of the sugars include monosaccharides, disaccharides, and tri- to hexa-saccharides as indicated below.
Examples of the monosaccharides include aldotetroses such as erythrose and threose, aldopentoses such as ribose, lyxose, xylose, and arabinose, aldohexoses such as allose, talose, gulose, glucose, altrose, mannose, galactose, and idose, ketotetroses such as erythrulose, ketopentoses such as xylulose and ribulose, ketohexoses such as psicose, fructose, sorbose, and tagatose. Examples of the disaccharides include α-diglucosides such as trehalose, kojibiose, nigerose, maltose, and isomaltose, β-diglucosides such as isotrehalose, sophorose, laminaribiose, cellobiose, and genthiobiose, and α,β-diglucosides such as neotrehalose, as well as lactose, sucrose, and isomaltulose (Palatinose). Examples of the trisaccharides include raffinose. Examples of the tri- to hexa-saccharide oligosaccharides include cyclic oligosaccharides such as fructooligosaccharide, galactooligosaccharide, xylooligosaccharide, isomaltooligosaccharide, chitin oligosaccharides, chitosan oligosaccharides, oligoglucosamine, dextrin, and cyclodextrin.

Examples of alcohols of the monosaccharides include tetritols such as erythritol, D-threitol, and L-threitol, pentitols such as D-arabinitol and xylitol, hexitols such as D-iditol, galactitol (dulcitol), D-glucitol (sorbitol), and mannitol, and cyclitols such as inositol. Examples of alcohols of the disaccharides include maltitol, lactitol, and reduced palatinose (isomalt). Examples of oligosaccharides include pentaerythritol and reduced maltose syrup.
In the sheet-form preparation of the present invention, the sugars or sugar alcohols may be substituted. Further, each of these may be used alone or two or more of these may be used in combination.

In order to easily dissolve the sheet-form preparation of the present invention in the mouth and not to greatly vary the viscosity of the solution in the production process, the sugars or sugar alcohols are preferably mono- to tri-saccharides or sugar alcohols thereof.
From the viewpoint of stabilization of allergenic protein from cedar pollen, D-sorbitol, D-mannitol, isomalt and sucrose are more preferable.

Examples of the sugar fatty acids include sorbitan fatty acid esters and sucrose fatty acid esters from the viewpoint of stabilization of allergenic protein from cedar pollen.
Examples of the sorbitan fatty acid esters include sorbitan monooleate, sorbitan trioleate, sorbitan sesquioleate, sorbitan cocoate, and polyoxyethylene sorbitan fatty acid esters.
Examples of the sucrose fatty acid esters include sucrose stearate, sucrose oleate, sucrose palmitate, sucrose myristate, sucrose behenate, sucrose erucate, and sucrose-mixed fatty acid esters.

The amount of the stabilizing agent is preferably 0.1 to 60 wt%, and more preferably 1 to 30 wt%, based on the total weight of the sheet-form preparation of the present invention. If the amount is less than 0.1 wt%, the storage stability of the allergenic protein from cedar pollen may not be sufficiently maintained during use. On the other hand, if the amount is in excess of 60 wt%, it may be difficult to control the physical properties of the sheet-form preparation by the added stabilizing agent.

The sheet-form preparation of the present invention contains water.
The water is an ingredient that has an effect of assisting dissolution of the gelatin.
In addition, controlling of the water content in the sheet-form preparation of the present invention enables to easily control the dissolution time of the sheet-form preparation. Consequently, the sheet-form preparation of the present invention is appropriate for intraoral, in particular sublingual, hyposensitization therapy, which requires control of the sensitization time.
In the present invention, the water content is preferably 1 to 60 wt%, and more preferably 10 to 50 wt%, based on the total weight of the sheet-form preparation. If the water content is less than 1 wt%, the solubility in the mouth cavity may be extremely poor, which may cause a problem during use. On the other hand, if the water content is in excess of 60 wt%, the storage stability at ordinary temperature regarding physical properties may be poor.

The sheet-form preparation of the present invention may further contain an antifoaming agent.
Examples of the antifoaming agent include, but not particularly limited to, sorbitan fatty acid esters and sucrose fatty acid esters, which have stabilization effects on the allergenic protein from cedar pollen. The sorbitan fatty acid esters and sucrose fatty acid esters may be those mentioned above. That is, sorbitan fatty acid esters and sucrose fatty acid esters function as stabilizing agents of the allergenic protein from cedar pollen, as well as function as antifoaming agents.

If necessary, the sheet-form preparation of the present invention may appropriately contain, as ingredients constituting the base material, a perfume, a flavoring substance, a sweetening agent, a colorant, an antiseptic, an antioxidant, a stabilizing agent, a surfactant, and the like, in addition to the ingredients mentioned above. These ingredients are not particularly limited and conventionally known ones may be used.

Since the sheet-form preparation of the present invention contains gelatin as mentioned above, it gels at ordinary temperature and can be easily dissolved at a temperature of about the body temperature in the mouth cavity. Further, since the preparation contains gelatin and a stabilizing agent thereof, it can stably maintain the allergenic protein from cedar pollen.
In addition, since the dissolution time of the sheet-form preparation of the present invention can be easily controlled by controlling the water content therein, it is suited for intraoral, in particular sublingual, hyposensitization therapy, which requires control of the sensitization time.
If the sheet-form preparation of the present invention is used for hyposensitization therapy, it shows higher versatility; that is, the preparation allows a patient to self-administer the allergenic protein from cedar pollen at home, has no residual feeling and has an excellent ability to prevent accidental ingestion, and is simple to administer for a caregiver, thereby widely increasing the QOL of patients and caregivers; furthermore, it enables to control intraoral, in particular sublingual, dissolution time, while allowing a cedar pollen extract in a solution state to be solidified, which has hitherto been difficult.

The sheet-form preparation of the present invention can be produced by, for instance, a method having the steps of: preparing a mixed solution by mixing water, gelatin, an allergenic protein from cedar pollen, and a stabilizing agent of the allergenic protein from cedar pollen; and forming a thin film using the mixed solution, wherein the water content in the obtained sheet-form preparation is adjusted by adjusting the amount of water to be added in the step of preparing a mixed solution or by nonthermally drying the thin film after the step of forming the thin film. Such a method for producing the sheet-form preparation of the present invention is also one aspect of the present invention.

In the step of preparing a mixed solution, for instance, gelatin and other additives are first dissolved in a predetermined amount of water at ordinary temperature or by heating and additives that do not dissolve are dispersed homogeneously to prepare a gelatin solution. Separately, an aqueous solution or a glycerol aqueous solution containing an allergenic protein from cedar pollen is prepared under low temperature and a stabilizing agent is added thereto to prepare a solution. Obviously, a cedar pollen extract may be used in lieu of the aqueous solution.
Next, the solution prepared is brought to ordinary temperature, and then it is stir-mixed with the gelatin solution separately prepared at a temperature of 28°C to 32°C to prepare a mixed solution.
If foaming occurs during the preparation of a mixed solution, it is adequate to leave the solution overnight or to perform vacuum or reduced pressure degassing.

In the step of forming a thin film, for instance, a predetermined amount of the mixed solution is dispensed into a plastic blister case of a desired size at a temperature of 28°C to 32°C, and cool-solidified immediately after the dispensation to form the thin film. In lieu of the dispensation method, a suitable amount of the mixed solution may be spread over a release film and cool-solidified to form the thin film, and then the film may be cut into a desired size.
The thin film formed in the present step preferably has a size equal to the sheet-form preparation of the present invention descried above.

In the production method for the sheet-form preparation of the present invention, the water content in the obtained sheet-form preparation is adjusted by adjusting the amount of water to be added in the step of preparing a mixed solution or by nonthermally drying the thin film after the step of forming a thin film.
That is, in the case of adjusting the water content in the step of preparing a mixed solution by adjusting the amount of water to be added, the sheet-form preparation of the present invention can be produced by forming the thin film described above.
Meanwhile, in the case of adjusting the water content after the step of forming a thin film by nonthermally drying the thin film, the sheet-form preparation of the present invention can be produced by drying the thin film described above.
Examples of the method for nonthermally drying the thin film include a method comprising a cold air drying step or a reduced pressure cool drying step.

The production method for the sheet-form preparation of the present invention is extremely useful for stabilization; for example, the film can be prepared under no heat, preferably at a temperature of 30°C or lower, with respect to allergenic proteins from cedar pollen having extremely low thermal stability; and long-term stability can be dramatically increased.
In addition, the obtained sheet-form preparation is preferably seal-packaged if necessary and turned into a product.

Since the sheet-form preparation of the present invention contains gelatin, it gels at ordinary temperature and can be easily dissolved at a temperature of about the body temperature in the mouth cavity. In addition, since the dissolution time of the film can be easily controlled by controlling the water content thereof, the sheet-form preparation of the present invention is suited for intraoral, in particular sublingual, hyposensitization therapy, which requires control of the sensitization time. Further, since the sheet-form preparation of the present invention contains gelatin and a stabilizing agent thereof, it can stably maintain the allergenic protein from cedar pollen.
In addition, the sheet-form preparation of the present invention is a sheet-form preparation containing an allergenic protein from cedar pollen with higher versatility; that is, if used for hyposensitization therapy, the preparation allows a patient to self-administer the allergenic protein from cedar pollen at home, has no residual feeling and has an excellent ability to prevent accidental ingestion, and is simple to administer for a caregiver, thereby widely increasing the QOL of patients and caregivers; furthermore, the preparation enables to adjust intraoral, in particular sublingual, dissolution time, while allowing a cedar pollen extract in a solution state to be solidified, which has hitherto been difficult.
Moreover, in the production method of the sheet-form preparation of the present invention, the film can be dried under no heat because gelatin, especially a water-soluble gelatin, is used. Thus, even though an allergenic protein from cedar pollen vulnerable to high temperature is used, a sheet-form preparation can be produced while reducing detrimental effects thereon.

The present invention will be described in detail with the following examples; however, the present invention is not limited to these examples.

### (Example 1)

To 29 parts by weight of purified water was added 1 part by weight of crystalline cellulose, and the cellulose was ultrasound-dissolved and dispersed therein. Added thereto was 10 parts by weight of fish (tilapia)-derived water-soluble gelatin (average molecular weight: approximately 100,000; hydroxyproline amount: approximately 8.6 mol%), which was dissolved at 30°C to 50°C and subjected to a shaker under a constant temperature of 28°C to 32°C to be turned into a gelatin solution.
Separately, 50 parts by weight of the therapeutic-use standardized allergen extract cedar pollen 2,000 JAU/mL was prepared, and 7 parts by weight of D-sorbitol and 3 parts by weight of PEG 4000 were dissolved therein at 2°C to 8°C. The mixture was heated to reach 25°C to 30°C, and then the whole was added to the gelatin solution prepared beforehand. They were immediately mixed at 28°C to 32°C, dispensed into 5-cm² plastic blister cases (Cryomold (square type) No. 3, Sakura Finetek Japan Co., Ltd.) in 2.2 g portions, and cool-solidified at 2°C to 8°C for one day and one night. Thereby, sheet-form preparations were obtained.

### (Examples 2 and 3)

Sheet-form preparations were obtained in the same manner as in Example 1 except that the compositions were those indicated in Table 1.
In Example 2, gelatin (fish-derived) (average molecular weight: approximately 100,000; hydroxyproline amount: approximately 8.6 mol%) was used; in Example 3, gelatin (pig-derived) A (average molecular weight: approximately 85,000; hydroxyproline amount: approximately 9.2 mol%) was used.

### (Comparative Example 1)

To 39 parts by weight of purified water was added 1 part by weight of crystalline cellulose, and the cellulose was ultrasound-dissolved and dispersed therein.
Separately, 50 parts by weight of the therapeutic-use standardized allergen extract cedar pollen 2,000 JAU/mL was prepared, and 7 parts by weight of D-sorbitol and 3 parts by weight of PEG 4000 were dissolved therein at 2°C to 8°C. The whole was added to the aqueous solution containing crystalline cellulose prepared beforehand. They were immediately mixed at room temperature, dispensed in 5-cm² plastic blister cases (Cryomold (square type) No. 3, Sakura Finetek Japan Co., Ltd.) in 2.2 g portions, and cool-solidified at 2°C to 8°C for one day and one night. Thereby, sheet-form preparations were obtained.

### (Example 4)

To 29 parts by weight of purified water was added 1 part by weight of crystalline cellulose, and the cellulose was ultrasound-dissolved and dispersed therein. Added thereto was 10 parts by weight of alkali-treated gelatin (pig-derived) (average molecular weight: 180,000; hydroxyproline amount: approximately 9.2 mol%), which was dissolved at 70°C to 80°C and subjected to a shaker under a constant temperature of 40°C to be turned into a gelatin solution.
Separately, 50 parts by weight of the therapeutic-use standardized allergen extract cedar pollen 2,000 JAU/mL was prepared, and 7 parts by weight of D-sorbitol and 3 parts by weight of PEG 4000 were dissolved therein at 2°C to 8°C. The mixture was heated to reach a temperature of 40°C, and then the whole was added to the gelatin solution prepared beforehand. They were immediately mixed at 40°C, dispensed in 5-cm² plastic blister cases (Cryomold (square type) No. 3, Sakura Finetek Japan Co., Ltd.) in 2.2 g portions, and cool-solidified at 2°C to 8°C for one day and one night. Thereby, sheet-form preparations were obtained.

### (Examples 5 to 7)

Sheet-form preparations were obtained in the same manner as in Example 4 except that the compositions were those indicated in Table 1.
In Example 5, an acid-treated gelatin (pig-derived) (average molecular weight: 100,000; hydroxyproline amount: approximately 9.2 mol%) was used; in Example 6, gelatin (pig-derived) B (average molecular weight: approximately 100,000; hydroxyproline amount: approximately 9.4 mol%) was used; and in Example 7, gelatin (cattle-derived) (average molecular weight: approximately 200,000; hydroxyproline amount: approximately 9.5 mol%) was used.

### (Examples 8 to 14)

Sheet-form preparations were obtained in the same manner as in Example 1 except that the compositions were those indicated in Table 2.

### (Comparative Example 2)

Sheet-form preparations were obtained in the same manner as in Example 1 except that the composition was that indicated in Table 2.

### (Examples 15 to 21)

Sheet-form preparations were obtained in the same manner as in Example 1 except that the compositions were those indicated in Table 3.

### (Examples 22 to 24)

Sheet-form preparations were obtained in the same manner as in Example 1 except that the compositions were those indicated in Table 4.

### (Examples 25 and 26)

Sheet-form preparations were obtained in the same manner as in Example 1 except that the compositions were those indicated in Table 5.

### (Example 27)

To 29 parts by weight of purified water was added 1 part by weight of crystalline cellulose, and the cellulose was ultrasound-dissolved and dispersed therein. Added thereto was 10 parts by weight of a fish (tilapia)-derived water-soluble gelatin (average molecular weight: approximately 100,000; hydroxyproline amount: approximately 8.6 mol%), which was dissolved at 30°C to 50°C and subjected to a shaker under a constant temperature of 28°C to 32°C to be turned into a gelatin solution.
Separately, 0.01 parts by weight of cedar pollen extract-Cj (LSL Co., Ltd.), 7 parts by weight of D-sorbitol, 3 parts by weight of PEG 4000, 25 parts by weight of glycerin, and 25 parts by weight of purified water were dissolved at 2°C to 8°C. The mixture was heated to reach 25°C to 30°C, and then the whole was added to the gelatin solution prepared beforehand. They were immediately mixed at 28°C to 32°C, dispensed in 5-cm² plastic blister cases (Cryomold (square type) No. 3, Sakura Finetek Japan Co., Ltd.) in 2.2 g portions, and cool-solidified at 2°C to 8°C for one day and one night. Thereby, sheet-form preparations were obtained.

### (Example 28)

Based on the composition indicated in Table 5, the mixed solution was prepared in the same manner as in Example 27. The solution was dispensed in 1-cm² plastic blister cases (Cryomold (square type) No. 1, Sakura Finetek Japan Co., Ltd.) in 0.44 g portions and cool-solidified at 2°C to 8°C for one day and one night. Thereby, sheet-form preparations were obtained.

### (Example 29)

To 29 parts by weight of purified water was added 1 part by weight of crystalline cellulose, and the cellulose was ultrasound-dissolved and dispersed therein. Added thereto was 10 parts by weight of a water-soluble gelatin (fish-derived), which was dissolved at 30°C to 50°C and subjected to a shaker under a constant temperature of 28°C to 32°C to be turned into a gelatin solution.
Separately, 0.002 parts by weight of cedar pollen extract-Cj (LSL Co., Ltd.) was prepared, and 25 parts by weight of glycerin and 25 parts by weight of purified water were mixed thereto. Further, 7 parts by weight of D-sorbitol and 3 parts by weight of PEG 4000 were dissolved therein at 2°C to 8°C. The mixture was heated to reach 25°C to 30°C, and then the whole was added to the gelatin solution prepared beforehand. They were immediately mixed at 28°C to 32°C, dispensed in 5-cm² plastic blister cases (Cryomold (square type) No. 3, Sakura Finetek Japan Co., Ltd.) in 2.2 g portions and cool-solidified at 2°C to 8°C for one day and one night. Thereby, sheet-form preparations were obtained.

### (Example 30)

Sheet-form preparations were obtained in the same manner as in Example 29 except that the composition was that indicated in Table 5.

### (Comparative Examples 3 and 4)

As indicated in Table 5, the liquid formulation of the cedar pollen was used in this state as the sample.

### (Comparative Example 5)

Sheet-form preparations were obtained in the same manner as in Example 29 except that the composition was that indicated in Table 5.

### [Test Methods]

The producibility during production and the allergen activity of the sheet-form preparations prepared in each example and comparative example were evaluated. In addition, the storage stability (residual allergen activity and sensory test (touch)) during one month storage at 5°C and 25°C was evaluated. The respective test methods are described below. Table 6 shows the results.

### (Producibility)

The ease of preparing the sheet-form preparation was evaluated with the four levels below. The evaluation was based on the possibility of preparation at near ordinary temperature.
4: The solution was easily prepared at near ordinary temperature.
3: The solution was prepared at near ordinary temperature, but the viscosity thereof slightly increased.
2: The viscosity of the solution increased at near ordinary temperature and problems in homogeneity occurred during mixing.
1: The solution gelled at near ordinary temperature and could not be handled.
In addition, those that did not gel at ordinary temperature were evaluated as 0.

### (Allergen activity test)

The allergen activity of Cryj1, one of the major allergens of cedar pollen, was measured using cedar pollen antigen ELISA Kit "Cryj1" (Seikagaku Biobusiness Corp.).
The principle of the measurement kit is a sandwich ELISA method that utilizes a monoclonal antibody (013, 053) specific to Cryj1, which is one of the Japanese Cedar (*Cryptomeria japonica*) pollen antigens, and the method allows specific Cryj1 measurement. To 100 µL of a reaction buffer solution included in the kit was added 20 µL of a standard solution or sample, and a primary reaction was carried out at ordinary temperature for 60 minutes. Then, 100 µL of HRP-labeled antibody solution was added thereto and a secondary reaction was carried out for 60 minutes. Added thereto was 100 µL of an enzyme substrate solution, and a reaction was carried out for 30 minutes at ordinary temperature while light was shielded. Finally, 100 µL of reaction stop solution was added thereto. Thereafter, the ultraviolet absorption intensity at 450 nm was measured. A calibration curve was determined based on the absorption intensity of the standard solution at each Cryj1 concentration, and the Cryj1 allergen activity (ng/mL) of each sample was measured according to the calibration curve. In the storage stability test, the initial value of the amount of Cryj1 added to each sample was taken as 100%, and the percentage in cryj1 allergen activity was determined after sampling for the storage stability test and immediately after production. The evaluation was carried out by scoring the percentage in Cryj1 allergen activity as described below.
5: exceeding 95% and 105% or lower
4: exceeding 90% and 95% or lower
3: exceeding 80% and 90% or lower
2: exceeding 50% and 80% or lower
1: 50% or lower

### (Storage stability test)

The prepared sheet-form preparations were stored in thermostatic chambers set to 5°C and 25°C. According to the evaluation methods for the respective test items, the test on the allergen activity remaining one month after the beginning of the storage and sensory test (touch) were carried out.

### (Sensory test (touch))

The sheet-form preparations which were cut in the examples and the comparative examples were actually touched with a finger for five seconds while drawing a circle, and discomfort was evaluated from the viewpoints of whether they are sticky and whether the finger becomes wet. The evaluation criteria were as follows:
4: The preparation was not sticky and the finger did not become wet;
3: The preparation was sticky or the finger became wet, slightly;
2: Discomfort was experienced regarding stickiness and finger wetness;
1: The preparation was considerably sticky and remained on the finger.
The samples were evaluated as 0 in the case that they were in liquid form.

**[Table 1]**

| Ingredient name | Examples [parts by weight] | | | | | | | Comparative example [parts by weight] |
|---|---|---|---|---|---|---|---|---|
| | 1 | 2 | 3 | 4 | 5 | 6 | 7 | 1 |
| Therapeutic-use standardized allergen extract cedar pollen 2,000 JAU/mL | 50 | 50 | 50 | 50 | 50 | 50 | 50 | 50 |
| Water-soluble gelatin (fish-derived) | 10 | - | - | - | - | - | - | - |
| Gelatin (fish-derived) | - | 10 | - | - | - | - | - | - |
| Gelatin (pig-derived) A | - | - | 10 | - | - | - | - | - |
| Alkali-treated gelatin (pig-derived) | - | - | - | 10 | - | - | - | - |
| Acid-treated gelatin (pig-derived) | - | - | - | - | 10 | - | - | - |
| Gelatin (pig-derived) B | - | - | - | - | - | 10 | - | - |
| Gelatin (cattle-derived) | - | - | - | - | - | - | 10 | - |
| D-sorbitol | 7 | 7 | 7 | 7 | 7 | 7 | 7 | 7 |
| PEG 4000 | 3 | 3 | 3 | 3 | 3 | 3 | 3 | 3 |
| Crystalline cellulose | 1 | 1 | 1 | 1 | 1 | 1 | 1 | 1 |
| Purified water | 29 | 29 | 29 | 29 | 29 | 29 | 29 | 39 |
| Dispensed amount [g/blister] | 2.2 | 2.2 | 2.2 | 2.2 | 2.2 | 2.2 | 2.2 | 2.2 |
| Size [cm²] | 5 | 5 | 5 | 5 | 5 | 5 | 5 | 5 |

**[Table 2]**

| Ingredient name | Examples [parts by weight] | | | | | | | Comparative example [parts by weight] |
|---|---|---|---|---|---|---|---|---|
| | 8 | 9 | 10 | 11 | 12 | 13 | 14 | 2 |
| Therapeutic-use standardized allergen extract cedar pollen 2,000 JAU/mL | 50 | 50 | 50 | 50 | 50 | 50 | 50 | 50 |
| Water-soluble gelatin (fish-derived) | 10 | 10 | 10 | 10 | 10 | 10 | 10 | 10 |
| D-sorbitol | 7 | - | - | - | - | - | - | - |
| Isomalt A | - | 7 | - | - | - | - | - | - |
| Isomalt B | - | - | 7 | - | - | - | - | - |
| Glucose | - | - | - | 7 | - | - | - | - |
| Raffinose | - | - | - | - | 7 | - | - | - |
| D-mannitol | - | - | - | - | - | 7 | - | - |
| Sucrose | - | - | - | - | - | - | 7 | - |
| Purified water | 30 | 30 | 30 | 30 | 30 | 30 | 30 | 37 |
| Dispensed amount [g/blister] | 2.2 | 2.2 | 2.2 | 2.2 | 2.2 | 2.2 | 2.2 | 2.2 |
| Size [cm²] | 5 | 5 | 5 | 5 | 5 | 5 | 5 | 5 |

**[Table 3]**

| Ingredient name | Examples [parts by weight] | | | | | | |
|---|---|---|---|---|---|---|---|
| | 15 | 16 | 17 | 18 | 19 | 20 | 21 |
| Therapeutic-use standardized allergen extract cedar pollen 2,000 JAU/mL | 50.0 | 50.0 | 50.0 | 50.0 | 50.0 | 50.0 | 50.0 |
| Water-soluble gelatin (fish-derived) | 10 | 10 | 10 | 10 | 10 | 10 | 10 |
| D-sorbitol | 7 | 7 | 7 | 7 | 7 | 7 | 7 |
| PEG 2000 | 3 | - | - | - | - | - | - |
| PEG 4000 | - | 3 | - | - | - | - | - |
| PEG 400 | - | - | 3 | - | - | - | |
| PEG 600 | - | - | - | 3 | - | - | - |
| PEG 6000 | - | - | - | - | 3 | - | - |
| PEG 20000 | - | - | - | - | - | 3 | - |
| Purified water | 30 | 30 | 30 | 30 | 30 | 30 | 30 |
| Dispensed amount [g/blister] | 2.2 | 2.2 | 2.2 | 2.2 | 2.2 | 2.2 | 2.2 |
| Size [cm²] | 5 | 5 | 5 | 5 | 5 | 5 | 5 |

**[Table 4]**

| Ingredient name | Examples [parts by weight] | | |
|---|---|---|---|
| | 22 | 23 | 24 |
| Therapeutic-use standardized allergen extract cedar pollen 2,000 JAU/mL | 50 | 50 | 50 |
| Water-soluble gelatin (fish-derived) | 10 | 10 | 10 |
| D-sorbitol | 7 | 7 | 7 |
| PEG 4000 | 3 | 3 | 3 |
| Crystalline cellulose | 1 | - | - |
| Powder cellulose | - | 1 | - |
| Purified water | 29 | 29 | 30 |
| Dispensed amount [g/blister] | 2.2 | 2.2 | 2.2 |
| Size [cm²] | 5 | 5 | 5 |

**[Table 5]**

| Ingredient name | Examples [parts by weight] | | | | | | Comparative examples [parts by weight] | | |
|---|---|---|---|---|---|---|---|---|---|
| | 25 | 26 | 27 | 28 | 29 | 30 | 3 | 4 | 5 |
| Therapeutic-use standardized allergen extract cedar pollen 200 JAU/mL | 50 | - | - | - | - | - | 100 | - | - |
| Therapeutic-use standardized allergen extract cedar pollen 2,000 JAU/mL | - | 50 | - | - | - | - | - | 100 | - |
| Cedar pollen extract-Cj | - | - | 0.01 | 0.01 | 0.002 | 1 | - | - | 0.02 |
| Water-soluble gelatin (fish-derived) | 10 | 10 | 10 | 10 | 10 | 10 | - | - | - |
| D-sorbitol | 7 | 7 | 7 | 7 | 7 | 7 | - | - | - |
| PEG 4000 | 3 | 3 | 3 | 3 | 3 | 3 | - | - | - |
| Crystalline cellulose | 1 | 1 | 1 | 1 | 1 | 1 | - | - | - |
| Glycerin | - | - | 25 | 25 | 25 | 24.5 | - | - | 50 |
| Purified water | 29 | 29 | 54 | 54 | 54 | 53.5 | - | - | 50 |
| Dispensed amount [g/blister] | 2.2 | 2.2 | 2.2 | 0.44 | 2.2 | 2.2 | - | | |
| Size [cm²] | 5 | 5 | 5 | 1 | 5 | 5 | - | - | - |

**[Table 6]**

| Examples | In preparation | | 5°C 1M Storage stability | | 25°C 1M Storage stability | | Total |
|---|---|---|---|---|---|---|---|
| | Producibility | Allergen activity | Allergen activity | Sense (touch) | Allergen activity | Sense (touch) | |
| Example 1 | 4 | 5 | 5 | 4 | 5 | 4 | 27 |
| Example 2 | 4 | 5 | 5 | 4 | 5 | 4 | 27 |
| Example 3 | 4 | 5 | 5 | 4 | 5 | 4 | 27 |
| Example 4 | 3 | 2 | 2 | 4 | 2 | 4 | 17 |
| Example 5 | 3 | 3 | 2 | 4 | 2 | 4 | 18 |
| Example 6 | 3 | 2 | 2 | 4 | 2 | 4 | 17 |
| Example 7 | 3 | 2 | 2 | 4 | 2 | 4 | 17 |
| Comparative Example 1 | 0 | 5 | 4 | 0 | 2 | 0 | 11 |
| Example 8 | 4 | 5 | 5 | 4 | 5 | 3 | 26 |
| Example 9 | 4 | 5 | 5 | 4 | 5 | 3 | 26 |
| Example 10 | 4 | 5 | 5 | 4 | 5 | 3 | 26 |
| Example 11 | 4 | 5 | 5 | 4 | 4 | 3 | 25 |
| Example 12 | 4 | 5 | 5 | 4 | 4 | 3 | 25 |
| Example 13 | 4 | 5 | 4 | 4 | 2 | 3 | 22 |
| Example 14 | 4 | 5 | 5 | 4 | 3 | 3 | 24 |
| Comparative Example 2 | 4 | 3 | 2 | 4 | 1 | 2 | 16 |
| Example 15 | 4 | 5 | 5 | 4 | 5 | 3 | 26 |
| Example 16 | 4 | 5 | 5 | 4 | 5 | 4 | 27 |
| Example 17 | 4 | 4 | 4 | 4 | 2 | 2 | 20 |
| Example 18 | 4 | 4 | 4 | 4 | 2 | 2 | 20 |
| Example 19 | 2 | 5 | 5 | 1 | 5 | 1 | 19 |
| Example 20 | 1 | 5 | 5 | 1 | 4 | 1 | 17 |
| Example 21 | 4 | 5 | 5 | 4 | 5 | 3 | 26 |
| Example 22 | 4 | 5 | 5 | 4 | 5 | 4 | 27 |
| Example 23 | 4 | 5 | 5 | 4 | 5 | 4 | 27 |
| Example 24 | 4 | 5 | 5 | 4 | 5 | 4 | 27 |
| Example 25 | 4 | 5 | 5 | 4 | 5 | 4 | 27 |
| Example 26 | 4 | 5 | 5 | 4 | 5 | 4 | 27 |
| Example 27 | 4 | 5 | 5 | 4 | 4 | 4 | 26 |
| Example 28 | 4 | 5 | 5 | 4 | 5 | 4 | 27 |
| Example 29 | 4 | 5 | 5 | 4 | 5 | 4 | 27 |
| Example 30 | 4 | 5 | 5 | 4 | 4 | 4 | 26 |
| Comparative Example 3 | 0 | 5 | 5 | 0 | 2 | 0 | 12 |
| Comparative Example 4 | 0 | 5 | 5 | 0 | 1 | 0 | 11 |
| Comparative Example 5 | 0 | 5 | 5 | 0 | 1 | 0 | 11 |

As shown in Table 6, the sheet-form preparations according to Examples 1 to 7, which had the same compositions except that their gelatin types were different, showed the sum of the evaluation items of 17 to 27; in contrast, the sheet-form preparation according to Comparative Example 1, which did not contain gelatin, showed the sum of the evaluation items of 11.
In addition, the sheet-form preparations according to Examples 8 to 14, which had the same compositions except that their stabilizing agent types were different, showed the sum of the evaluation items of 22 to 26; in contrast, the sheet-form preparation according to Comparative Example 2, which did not contain a stabilizing agent, showed the sum of the evaluation items of 16.
In addition, the sheet-form preparations according to Examples 15 to 30, in which the types of the other edible polymers, cellulose, and cedar pollen extract, the presence/absence of glycerin, and the size of the sheet-form preparation were different, showed the sum of the evaluation items of 17 to 27.
In addition, the Comparative Examples 3 to 5, in each of which the liquid preparation of the cedar pollen extract as it is was used as a sample, showed the sum of the evaluation items of 11 to 12.

### (Example 31)

The sheet-form preparation prepared in Example 1 (water content: 54 wt%) was placed in a desiccator lined with silica gel and stored under 2°C to 8°C for one day to extract moisture. Thereby, a sheet-form preparation with a water content of 45 wt% was obtained. The water content was determined by the change in weight.

### (Example 32)

The sheet-form preparation prepared in Example 1 (water content: 54 wt%) was placed in a desiccator lined with silica gel and stored under 2°C to 8°C below for two days to extract moisture. Thereby, a sheet-form preparation with a water content of 35 wt% was obtained. The water content was determined by the change in weight.

The sheet-form preparations according to Examples 1, 31, and 32 were subjected to the following intraoral solubility test. Table 7 shows the results.

### (Intraoral solubility test)

The test was carried out according to the disintegration test described in the Japanese Pharmacopoeia 15th revision. Distilled water was introduced in a 1000-mL low-form beaker and the test was carried out at 37 ± 2°C, under conditions where the test container was raised and lowered with an amplitude of 53 to 57 mm at 29 to 32 rounds in one minute. A sheet-form preparation was introduced in the test container, and the test was started under the conditions described above. The time from the beginning of the test to when the sheet-form preparation was completely dissolved and disappeared from the test container was taken as the intraoral dissolution time.

| Examples | Water content [wt%] | Intraoral dissolution time [second] |
|---|---|---|
| 1 | 54 | 42 |
| 31 | 45 | 78 |
| 32 | 35 | 137 |

As shown in Table 7, it was demonstrated that the intraoral dissolution time can be controlled by controlling the water content of the sheet-form preparation.

Since the sheet-form preparation containing an allergenic protein from cedar pollen of the present invention contains gelatin, it gels at ordinary temperature and can be easily dissolved at a temperature of about body temperature in the mouth cavity. Further, since the sheet-form preparation of the present invention contains gelatin and a stabilizing agent thereof, it can stably maintain the allergenic protein from cedar pollen.
In addition, since the dissolution time of the film can be easily controlled by controlling the water content thereof, the sheet-form preparation of the present invention is suited for intraoral, in particular sublingual, hyposensitization therapy, which requires control of the sensitization time.
Moreover, the sheet-form preparation can be cold dried because gelatin, especially a water-soluble gelatin, is used. Thus, even though an allergenic protein from cedar pollen vulnerable to high temperature is used, a sheet-form preparation can be produced while reducing detrimental effects thereon.

## Claims

1. A sheet-form preparation, comprising:
water;
gelatin;
an allergenic protein from cedar pollen; and
a stabilizing agent of the allergenic protein from cedar pollen.

2. The sheet-form preparation according to claim 1, which is provided for intraoral hyposensitization therapy use.

3. The sheet-form preparation according to claim 1 or 2,
wherein the gelatin has a property of not gelling at 32°C when turned into an aqueous solution at a concentration of 10 wt%.

4. The sheet-form preparation according to claim 1, 2 or 3,
wherein the allergenic protein from cedar pollen is provided in a liquid or solid form containing this protein.

5. The sheet-form preparation according to claim 1, 2, 3 or 4,
wherein the amount of the gelatin is 2 to 40 wt% based on the total weight.

6. The sheet-form preparation according to claim 1, 2, 3, 4 or 5, which has a thickness within the range of 30 to 5000 µm.

7. The sheet-form preparation according to claim 1, 2, 3, 4, 5 or 6, which has a planar surface area within the range of 0.5 to 6.0 cm²

8. The sheet-form preparation according to claim 1, 2, 3, 4, 5, 6 or 7,
wherein the stabilizing agent contains at least one selected from the group consisting of sugars, sugar alcohols, and sugar fatty acids.

9. The sheet-form preparation according to claim 1, 2, 3, 4, 5, 6, 7 or 8, further comprising polyethylene glycol or a derivative thereof.

10. The sheet-form preparation according to claim 1, 2, 3, 4, 5, 6, 7, 8 or 9, further comprising crystalline cellulose.

11. A method for producing the sheet-form preparation according to claim 1, the method comprising the steps of:
preparing a mixed solution by mixing water, gelatin, an allergenic protein from cedar pollen, and a stabilizing agent of the allergenic protein from cedar pollen; and
forming a thin film using the mixed solution,
wherein a water content in the obtained sheet-form preparation is adjusted by adjusting the amount of water to be added in the step of preparing a mixed solution or by nonthermally drying the thin film after the step of forming a thin film.
